# EUROPÄISCHE PATENTSCHRIFT

(11) **EP 0 565 952 B1**
(45) Veröffentlichungstag und Bekanntmachung des Hinweises auf die Patenterteilung: **11.09.1996**
(21) Anmeldenummer: 93105335.9
(22) Anmeldetag: 31.03.1993
(51) Int. Cl.: B01J 23/60, B01J 37/34, C07C 67/055

(54) **Trägerkatalysator, Verfahren zu dessen Herstellung, sowie dessen Verwendung zur Herstellung von Vinylacetat**
Supported catalyst, preparation process thereof and use thereof for preparing vinyl acetate
Catalyseur sur support, procédé pour sa préparation, ainsi que son utilisation pour préparer l'acétate de vinyle

(30) Priorität: 08.04.1992 DE 4211780
(43) Veröffentlichungstag der Anmeldung: 20.10.1993
(73) Patentinhaber: HOECHST AKTIENGESELLSCHAFT, 65926 Frankfurt am Main (DE)
(72) Erfinder: Wirtz, Peter, Dr., W-6240 Königstein/Ts (DE); Wunder, Friedrich, Dr., W-6234 Hattersheim/M (DE); Wörner, Karl-Fred, Dr., W-6236 Eschborn (DE)

(56) Entgegenhaltungen:
- EP-A- 0 154 408
- DE-A- 2 745 174
- FR-A- 2 223 355
- GB-A- 1 267 354

## Beschreibung

Es ist bekannt, Vinylacetat in der Gasphase aus Ethylen, Essigsäure und Sauerstoff herzustellen; die für diese Synthese verwendeten Trägerkatalysatoren enthalten Palladium und ein Alkalielement, vorzugsweise Kalium. Als weitere Zusätze werden Cadmium, Gold oder Barium verwendet.

Bei den Pd/K/Au-Katalysatoren sind die beiden Edelmetalle im allgemeinen in Form einer Schale auf den Träger aufgebracht; die Herstellung erfolgt durch Imprägnierung und anschließende Fällung der Metallsalze mittels alkalischer Verbindungen (US-PS 4 048 096, US-PS 3 775 342).

Bei Pd/K/Ba-Katalysatoren werden die Metallsalze durch Tränken, Aufsprühen, Aufdampfen, Tauchen oder Ausfällen aufgebracht (Deutsche Patentanmeldung P 41 20 491.3). Dieselben Methoden sind bei Pd/K/Cd-Katalysatoren bekannt (US-PS 4 902 823; US-PS 3 393 199, US-PS 4 668 819); weiter wurde hier die Herstellung eines Schalenkatalysators beschrieben, wobei ein spezielles Trägermaterial vor der Imprägnierung mit einer Säure gewaschen und nach der Imprägnierung mit einer Base behandelt wird (Deutsche Patentanmeldung P 41 20 492.1).

Überraschenderweise wurde nun gefunden, daß man verbesserte Pd/K/Au-, Pd/K/Ba- bzw. Pd/K/Cd-Katalysatoren erhält, wenn man eine Lösung entsprechender Metallsalze mittels Ultraschall zerstäubt und dann in derart beschränkten Mengen und innerhalb einer derart beschränkten Zeit auf die Trägerteilchen aufbringt und mit deren Trocknung beginnt, daß die katalytisch wirksamen Metallsalze nicht bis zum Kern in die Trägerteilchen eindringen können, sondern nur in einen mehr oder weniger großen äußeren Teil. Das heißt, die durch Ultraschall zerstäubte Lösung wird so aufgebracht, daß Schalenkatalysatoren entstehen. Überraschenderweise zeigen diese eine deutlich verbesserte Selektivität sowie Leistung bzw. spezifische Leistung bei der Herstellung von Vinylacetat, die nicht nur die entsprechenden Werte der (bis in den Kern) durchimprägnierten Katalysatoren gemäß US-PS 4 902 823 übertrifft, sondern auch die Werte der durch andere Methoden hergestellten Schalenkatalysatoren (US-PS 4 048 096, US-PS 3 775 342, Deutsche Patentanmeldung P 41 20 492.1).

Ein Gegenstand der Erfindung ist ein Verfahren zur Herstellung eines Palladium, Kalium und Cadmium enthaltenden Trägerkatalysators, dadurch gekennzeichnet, daß man Salze des Palladiums, Kaliums und Cadmiums in einem geeigneten Lösungsmittel löst, die Lösung durch Ultraschall zerstäubt und mit der zerstäubten Lösung das Trägermaterial ein- oder mehrmals imprägniert und nach jeder Imprägnierung trocknet, wobei das Lösungsvolumen bei jeder Imprägnierung mindestens 5 bis höchstens 60 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 5 bis höchstens 60 % des Volumens der Trägerteilchen die genannten Salze enthält. Weitere Gegenstände der Erfindung sind ein auf diese Weise hergestellter Trägerkatalysator, sowie dessen Verwendung zur Herstellung von Vinylacetat aus Ethylen, Essigsäure und Sauerstoff in der Gasphase.

Vorzugsweise beträgt das Lösungsvolumen bei jeder Imprägnierung mit den Pd-, K- und Cd-Salzen mindestens 5 bis höchstens 50 % des Porenvolumens des Trägermaterials - insbesondere mindestens 15 bis höchstens 40 % des Porenvolumens - wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 5 bis höchstens 50 % des Volumens der Trägerteilchen die genannten Salze enthält - insbesondere eine Schale von mindestens 15 bis höchstens 40 % des Volumens der Trägerteilchen.

Ein weiterer Gegenstand der Erfindung ist ein Verfahren zur Herstellung eines Palladium, Kalium und Barium enthaltenden Trägerkatalysators, dadurch gekennzeichnet, daß man Salze des Palladiums, Kaliums und Bariums in einem geeigneten Lösungsmittel löst, die Lösung durch Ultraschall zerstäubt und mit der zerstäubten Lösung das Trägermaterial ein- oder mehrmals imprägniert und nach jeder Imprägnierung trocknet, wobei das Lösungsvolumen bei jeder Imprägnierung mindestens 15 bis höchstens 80 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 15 bis höchstens 80 % des Volumens der Trägerteilchen die genannten Salze enthält. Weitere Gegenstände der Erfindung sind ein auf diese Weise hergestellter Trägerkatalysator, sowie dessen Verwendung zur Herstellung von Vinylacetat aus Ethylen, Essigsäure und Sauerstoff in der Gasphase.

Vorzugsweise beträgt das Lösungsvolumen bei jeder Imprägnierung mit den Pd-, K- und Ba-Salzen mindestens 20 bis höchstens 80 % des Porenvolumens des Trägermaterials - insbesondere mindestens 25 bis höchstens 40 % des Porenvolumens - wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 20 bis höchstens 80 % des Volumens der Trägerteilchen die genannten Salze enthält - insbesondere eine Schale von mindestens 25 bis höchstens 40 % des Volumens der Trägerteilchen.

Ein weiterer Gegenstand der Erfindung ist ein Verfahren zur Herstellung eines Palladium, Kalium und Gold enthaltenden Trägerkatalysators, dadurch gekennzeichnet, daß man Salze des Palladiums, Kaliums und Golds in einem geeigneten Lösungsmittel löst, die Lösung durch Ultraschall zerstäubt und mit der zerstäubten Lösung das Trägermaterial ein- oder mehrmals imprägniert und nach jeder Imprägnierung trocknet, wobei das Lösungsvolumen bei jeder Imprägnierung mindestens 5 bis höchstens 80 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 5 bis höchstens 80 % des Volumens der Trägerteilchen die genannten Salze enthält. Weitere Gegenstände der Erfindung sind ein auf diese Weise hergestellter Trägerkatalysator, sowie dessen Verwendung zur Herstellung von Vinylacetat aus Ethylen, Essigsäure und Sauerstoff in der Gasphase.

Vorzugsweise beträgt das Lösungsvolumen bei jeder Imprägnierung mit den Pd-, K- und Au-Salzen mindestens 10 bis höchstens 60 % des Porenvolumens des Trägermaterials - insbesondere mindestens 15 bis höchstens 50 % des Porenvolumens - wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 10 bis höchstens 60 % des Volumens der Trägerteilchen die genannten Salze enthält - insbesondere eine Schale von mindestens 15 bis höchstens 50 % des Volumens der Trägerteilchen.

Als Träger werden inerte Materialien, wie Siliciumdioxid, Aluminiumoxid oder Gemische dieser Oxide in Form von Kugeln, Tabletten, Ringen, Sternen oder anderen Formkörpern benutzt; der Durchmesser, bzw. die Länge und Dicke der Trägerteilchen liegt im allgemeinen zwischen 4 und 15 mm.

Die Oberfläche der Träger liegt, gemessen mit der BET-Methode, im allgemeinen bei 40 bis 300 m²/g; das Porenvolumen liegt im allgemeinen bei 0,5 bis 1,0 ml/g.

Die Metallgehalte der fertigen Katalysatoren haben folgende Werte:

Der Palladium-Gehalt der Pd/K/Cd- und der Pd/K/Ba-Katalysatoren beträgt im allgemeinen 0,6 bis 3,5 Gew.-%, vorzugsweise 0,8 bis 3,0 Gew.-%, insbesondere 1,0 bis 2,5 Gew.-%. Der Palladium-Gehalt der Pd/K/Au-Katalysatoren beträgt im allgemeinen 0,5 bis 2,0 Gew.-%, vorzugsweise 0,6 bis 1,5 Gew.-%.

Der Kalium-Gehalt aller drei Katalysatoren-Arten beträgt im allgemeinen 0,5 bis 4,0 Gew.-%, vorzugsweise 1,5 bis 3,0 Gew.-%.
Der Cadmium-Gehalt der Pd/K/Cd-Katalysatoren beträgt im allgemeinen 0,1 bis 2,5 Gew.-%, vorzugsweise 0,4 bis 2,0 Gew.-%.
Der Barium-Gehalt der Pd/K/Ba-Katalysatoren beträgt im allgemeinen 0,1 bis 2,0 Gew.-%, vorzugsweise 0,2 bis 1,0 Gew.-%.
Der Gold-Gehalt der Pd/K/Au-Katalysatoren beträgt im allgemeinen 0,2 bis 1,0 Gew.-%, vorzugsweise 0.3 bis 0.8 Gew.-%.

Als Salze sind alle Salze von Palladium, Cadmium, Barium, Gold und Kalium geeignet, die löslich sind und keine für den Katalysator giftigen Bestandteile, wie z.B. Schwefel enthalten; bevorzugt sind die Acetate und die Chloride. Dabei muß aber im Fall der Chloride sichergestellt werden, daß die Chloridionen vor dem Einsatz des Katalysators entfernt werden. Dies geschieht durch Auswaschen des dotierten Trägers, z.B. mit Wasser, nachdem Palladium und ggf. Gold in eine unlösliche Form überführt wurden, etwa durch Reduktion.

Als Lösungsmittel sind alle Verbindungen geeignet, in denen die gewählten Salze löslich sind und die nach der Imprägnierung leicht wieder durch Trocknung zu entfernen sind. Geeignet sind für die Acetate vor allem unsubstituierte Carbonsäuren mit 2 bis 10 Kohlenstoffatomen, wie Essigsäure, Propionsäure, n- und iso-Buttersäure und die verschiedenen Valeriansäuren. Wegen ihrer physikalischen Eigenschaften und auch aus wirtschaftlichen Gründen wird Essigsäure unter den Carbonsäuren bevorzugt. Für die Chloride ist vor allem Wasser geeignet. Die zusätzliche Verwendung eines weiteren Lösungsmittel ist dann zweckmäßig, wenn die Salze in der Essigsäure oder im Wasser nicht genügend löslich sind. Als zusätzliche Lösungsmittel kommen diejenigen in Betracht, die inert und mit Essigsäure bzw. Wasser mischbar sind. Genannt seien als Zusätze für Essigsäure Ketone wie Aceton und Acetylaceton, ferner Ether wie Tetrahydrofuran oder Dioxan, aber auch Kohlenwasserstoffe wie Benzol.

Die Lösung der Salze wird erfindungsgemäß durch Ultraschall zerstäubt.

Vorzugsweise beträgt die Frequenz des Ultraschalls 30 bis 200 kHz, vorzugsweise 80 bis 200 kHz. Geeignete Geräte für diese Zerstäubung sind unter dem Namen "Ultraschall-Flüssigkeitszerstäuber" oder "Ultrasonic Atomizer" oder "Ultrasonic Nebulizer" im Handel erhältlich (z. B. von den Herstellern Siemens AG, Erlangen oder Lechler, Metzingen, Deutschland).

Die Lösung der Salze sollte bei der Ultraschall-Zerstäubung eine Temperatur haben, die hoch genug ist, um ein Ausfallen der Salze während des Aufbringens auf den Träger zu verhindern. Die Temperatur sollte aber im allgemeinen nicht wesentlich über 70° C liegen, um eine zu starke Verdunstung des Lösungsmittels zu verhindern.

Die Dauer der Imprägnierung muß einerseits so kurz sein, daß eine Schale entsteht, die bei den Pd/K/Cd-Katalysatoren mindestens 5 bis höchstens 60 % des Porenvolumens der Trägerteilchen ausmacht, bzw. mindestens 5 bis höchstens 80 % bei den Pd/K/Au-Katalysatoren, sowie mindestens 15 bis höchstens 80 % bei den Pd/K/Ba-Katalysatoren. Andererseits muß die Dauer der Imprägnierung ausreichend lang sein, um eine gleichmäßige Schalendicke aller Trägerteilchen sicherzustellen. Die optimale Dauer ist abhängig von der Menge der Salze, von der Menge des Lösungsmittels und von der Menge des zu imprägnierenden Trägermaterials; sie läßt sich jedoch durch Vorversuche leicht feststellen. Eine geeignete Methode, um die erzielte Verteilung der Schalendicken festzustellen, besteht im Aufschneiden einer repräsentativen Zahl von Trägerteilchen und Vermessen der Schalendicken unter dem Mikroskop. Hierbei sollten im allgemeinen weniger als 5 % der Teilchen eine Schalendicke haben, die um mehr als 15 % vom Durchschnitt abweicht.

Bei der Imprägnierung empfiehlt es sich, auf eine gleichmäßige Durchmischung der Trägerteilchen zu achten, beispielsweise indem man in einem sich drehenden Kolben oder einer Mischtrommel arbeitet. Die Drehgeschwindigkeit muß einerseits groß genug sein, um eine gute Durchmischung zu gewährleisten, andererseits darf sie nicht so hoch sein, daß es zu wesentlichem Abrieb am Trägermaterial kommt.

Für eine Chargengröße von 1 Liter Trägermaterial hat sich in einem 10 l-Kolben eine Drehgeschwindigkeit von 100 bis 200 Umdrehungen/Minute bewährt.

Bei der Trocknung des mit der Lösung der aktiven Katalysatorenkomponenten imprägnierten Trägers empfiehlt es sich, die Temperatur an die Art der eingesetzten Metallsalze anzupassen. Bei den Acetaten, die häufig zur Herstellung von Pd/K/Cd- oder Pd/K/Ba-Katalysatoren eingesetzt werden, wird die Trocknung vorzugsweise unter vermindertem Druck durchgeführt. Die Temperatur sollte dabei im allgemeinen 50 bis 80° C, vorzugsweise 50 bis 70° betragen. Weiterhin empfiehlt es sich im allgemeinen, die Trocknung in einem Inertgasstrom, beispielsweise in einem Stickstoff- oder Kohlendioxidstrom, vorzunehmen. Bei den im allgemeinen mit den entsprechenden Chloriden imprägnierten Pd/K/Au-Katalysatoren kann die Trocknung dagegen in einem Heißluftstrom bei 100 bis 150° C durchgeführt werden. Der Lösungsmittelrestgehalt nach der Trocknung sollte bei allen drei Katalysator-Arten vorzugsweise weniger als 6 Gew.-% betragen.

Falls eine Reduktion des Palladiumsalzes und ggf. des Goldsalzes durchgeführt wird, was manchmal nützlich ist, so kann diese mit einem gasförmigen Reduktionsmittel durchgeführt werden. Die Reduktionstemperatur liegt im allgemeinen zwischen 40 und 260° C, vorzugsweise zwischen 70 und 200° C. Im allgemeinen ist es zweckmäßig, für die Reduktion ein mit Inertgas verdünntes Reduktionsmittel zu verwenden, das 0,01 bis 50 Vol-%, vorzugsweise 0,5 bis 20 Vol-% Reduktionsmittel enthält. Als Inertgas kann beispielsweise Stickstoff, Kohlendioxid oder ein Edelgas verwendet werden. Als Reduktionsmittel kommen beispielsweise Wasserstoff, Methanol, Formaldehyd, Ethylen, Propylen, Isobutylen, Butylen oder andere Olefine in Frage. Die Menge des Reduktionsmittels richtet sich nach der Palladiummenge und ggf. der Goldmenge; das Reduktionsäquivalent soll mindestens das 1- bis 1,5-fache des Oxydationsäquivalents betragen, jedoch schaden größere Mengen Reduktionsmittel nicht. Eine solche Reduktion wird im Anschluß an die Trocknung vorgenommen.

Die Herstellung des Vinylacetats erfolgt im allgemeinen durch Leiten von Essigsäure, Ethylen und Sauerstoff oder Sauerstoff enthaltenden Gasen bei Temperaturen von 100 bis 220° C, vorzugsweise 120 bis 200° C, und bei Drucken von 1 bis 25 bar, vorzugsweise 1 bis 20 bar, über den fertigen Katalysator, wobei nicht umgesetzte Komponenten im Kreis geführt werden können. Zweckmäßig hält man die Sauerstoffkonzentration unter 10 Vol-% (bezogen auf das essigsäurefreie Gasgemisch). Unter Umständen ist jedoch auch eine Verdünnung mit inerten Gasen wie Stickstoff oder Kohlendioxid vorteilhaft. Besonders Kohlendioxid eignet sich zur Verdünnung, da es in geringen Mengen während der Reaktion gebildet wird.

Mit Hilfe der erfindungsgemäßen Katalysatoren ist eine selektivere Verfahrensdurchführung bzw. eine Kapazitätserweiterung möglich. Zur Kapazitätserweiterung bietet es sich an, die Reaktionsbedingungen (z. B. Druck, Temperatur, Durchsatz, Sauerstoffkonzentration) gegenüber den bekannten Katalysatoren unverändert zu halten und mehr Vinylacetat pro Reaktorvolumen und Zeit herzustellen. Dadurch wird die Aufarbeitung des erhaltenen Rohvinylacetats erleichtert, da der Vinylacetatgehalt im Reaktorausgangsgas höher ist, was weiterhin zu einer Energieersparnis im Aufarbeitungsteil führt. Eine geeignete Aufarbeitung wird z. B. in der US-PS 5 066 365 beschrieben.

Hält man dagegen die Anlagenkapazität konstant, so kann man die Reaktionstemperatur senken und dadurch bei gleicher Gesamtleistung die Reaktion selektiver durchführen, wobei Edukte eingespart werden. Dabei wird weiterhin die Menge des als Nebenprodukt entstehenden und daher auszuschleusenden Kohlendioxids und der mit dessen Ausschleusung verbundene Verlust an mitgeschlepptem Ethylen geringer. Darüberhinaus führt diese Fahrweise zu einer Verlängerung der Katalysatorstandzeit.

Die folgenden Beispiele sollen die Erfindung erläutern.

Als Katalysatorträger wurde SiO₂ in Form von Tabletten mit einem Durchmesser und einer Höhe von jeweils 6 mm verwendet. Die Tabletten waren aus ®Aerosil-Pulver mit Hilfe von Magnesiumstearat als Bindemittel gemäß DE-OS 3 912 504 gepreßt worden. Die Oberfläche des Trägers betrug 120 m²/g, sein Porenvolumen 0,784 ml/g und sein Schüttgewicht 500 g/l.

### Vergleichsbeispiel 1

1 l Kieselsäureträger wurden mit einer Lösung von 24,3 g Palladiumacetat, 21,3 g Cadmiumacetat und 23,8 g Kaliumacetat in 392 ml Eisessig (Lösungsvolumen = 100% des Porenvolumens des Trägers) bei 60° C getränkt. Anschließend wurde in einem Trockenschrank bei 200 mbar unter Stickstoff bis zu einem Restgehalt an Essigsäure von 6 Gew.-% getrocknet; die Trocknungstemperatur betrug 65° C. Der fertige Katalysator enthielt 2,3 Gew.-% Pd, 1,8 Gew.-% Cd und 1,9 Gew.-% K.

Es wurden 50 ml dieses Katalysators in ein Reaktionsrohr von 8 mm Innendurchmesser und einer Länge von 1,5 m eingefüllt. Dann wurde bei einem Druck von 8 bar (Reaktoreingang) und einer Katalysatortemperatur von 150° C das umzusetzende Gas über den Katalysator geleitet. Dieses Gas bestand aus 27 Vol.-% Ethylen, 55 Vol.-% Stickstoff, 12 Vol.-% Essigsäure und 6 Vol.-% Sauerstoff. Die Ergebnisse sind aus der Tabelle 1 ersichtlich.

### Vergleichsbeispiel 2

Die Katalysatorherstellung erfolgte wie in Vergleichsbeispiel 1, außer daß anstelle von Cadmiumacetat jetzt 4,0 g Bariumacetat aufgebracht wurden. Der fertige Katalysator enthielt 2,3 Gew.-% Pd, 0,4 Gew.-% Ba und 1,9 Gew.-% K.

Die Austestung erfolgte wie in Vergleichsbeispiel 1; die Ergebnisse sind aus der Tabelle 1 ersichtlich.

### Vergleichsbeispiel 3

1 l Kieselsäureträger wurden gemäß der deutschen Patentanmeldung P 41 20 492.1 zur Entfernung des die Schalenbildung störenden Bindemittels mit 10 %iger Salzsäure und dann mit Wasser gewaschen und getrocknet. Anschließend wurde der Träger mit einer Lösung von 12,5 g Palladiumchlorid und 16,5 g Cadmiumchlorid in 392 ml Wasser imprägniert. Nach der Trocknung mit Heißluft bei 150° C wurden (zur Erzeugung einer Schale durch Ausfällung von Palladium und Cadmium) 7,5 g NaOH, gelöst in 392 ml Wasser, zugegeben. Anschließend wurde für 6 Stunden gerührt und für 16 Stunden bei Raumtemperatur stehen gelassen. Nachdem der Träger mit Wasser chloridfrei gewaschen und mit Heißluft bei 150°C getrocknet worden war, wurden 37,6 g Kaliumacetat in 392 ml Wasser aufgebracht. Nach der Trocknung mit Heißluft bei 150°C enthielt der Katalysator 1,5 Gew.-% Pd, 2,0 Gew.-% Cd und 3,0 Gew.-% K. Die Dicke der durch die Behandlung mit Natronlauge erzeugten Schale betrug 1,5 bis 1,7 mm. Die Austestung erfolgte wie in Vergleichsbeispiel 1. Die Ergebnisse sind aus der Tabelle 1 ersichtlich.

### Vergleichsbeispiel 4

Die Katalysatorherstellung erfolgte wie in Vergleichsbeispiel 3, außer daß anstelle von Cadmiumchlorid jetzt 4,0 g Tetrachlorogoldsäure und anstelle von Palladiumchlorid 13,8 g Natriumchloropalladat eingesetzt werden und daß die Menge an NaOH jetzt 5,5 g und die an Kaliumacetat 35,1 g betrug.

Der fertige Katalysator enthielt 1,0 Gew.-% Pd, 0,4 Gew.-% Au und 2,8 Gew.-% K. Die Schalendicke betrug 1,3 bis 1,6 mm.

Die Austestung erfolgte in einem Berty-Reaktor bei 152°C mit einem Gasgemisch aus 8 Vol.-% O₂, 37,5 Vol.-% C₂H₄, 15,7 Vol.-% HOAc und 38,8 Vol.-% N₂; die Ergebnisse sind aus der Tabelle 1 ersichtlich.

### Beispiel 1a

Bei 65°C wurden 16 g Palladiumacetat, 25 g Cadmiumacetat und 25,3 g Kaliumacetat in 58,8 ml Essigsäure (Lösungsvolumen = 15 % des Porenvolumens) gelöst und die hochviskose Lösung in eine auf 65°C vorgewärmte Vorlage eingefüllt. 1 l Katalysatorträger wurden in einer temperierbaren Mischtrommel ebenfalls auf 65°C erwärmt und mit einer Umdrehungszahl von 150 Umdrehungen/Minute durchmischt. Innerhalb einer Stunde wurde die Imprägnierlösung mittels eines Ultraschall-Zerstäubers (100 kHz) auf den Katalysatorträger aufgebracht.

Anschließend wurde getrocknet wie im Vergleichsbeispiel 1. Der fertige Katalysator enthielt 1,4 Gew.-% Pd, 1,8 Gew.-% Cd und 1,9 Gew.-% K. Die Schalendicke betrug 0,5 mm.

Die Austestung erfolgte wie in Vergleichsbeispiel 1. Die Ergebnisse sind aus der Tabelle 2 ersichtlich.

### Beispiel 1b

Die Katalysatorherstellung erfolgte wie in Beispiel 1a, außer daß statt 16 g Palladiumacetat und 60 ml Essigsäure jetzt 25,3 g Palladiumacetat und 137,2 ml Essigsäure (Lösungsvolumen = 35 % des Porenvolumens) eingesetzt wurden.

Der Katalysator enthielt 2,3 Gew.-% Palladium, 1,8 Gew.-% Cd und 1,9 Gew.-% K. Die Schalendicke betrug 0,8 mm.

Die Austestung erfolgte wie in Vergleichsbeispiel 1. Die Ergebnisse sind aus der Tabelle 2 ersichtlich.

### Beispiel 2

Die Katalysatorherstellung erfolgte wie in Beispiel 1b, außer daß anstelle von Cadmiumacetat jetzt 4,3 g Bariumacetat eingesetzt wurden. Der fertige Katalysator enthielt 2,3 Gew.-% Pd, 0,4 Gew.-% Ba und 1,9 Gew.-% K, die Schalendicke betrug 0,8 mm.

Die Austestung erfolgte wie im Vergleichsbeispiel 1. Die Ergebnisse sind aus der Tabelle 2 ersichtlich.

### Beispiel 3

13,8 g Natriumchloropalladat und 4,0 g Tetrachlorogoldsäure wurden in 78,4 ml Wasser (Lösungsvolumen = 20 % des Porenvolumens) gelöst. Die Lösung wurde bei Raumtemperatur innerhalb einer Stunde mittels eines Ultraschall-Zerstäubers (100 kHz) auf 1 l Katalysatorträger aufgebracht; anschließend wurde im Heißluftstrom bei 150°C getrocknet. Danach wurde zur Ausfällung von Palladium und Cadmium analog zu Vergleichsbeispiel 3 eine Lösung von 5,5 g NaOH in 78,4 ml Wasser mit dem Ultraschall-Zerstäuber auf den imprägnierten Träger aufgebracht. Danach wurde er entsprechend Vergleichsbeispiel 3 chloridfrei gewaschen und getrocknet. Anschließend wurde er mit H₂ reduziert, mit 35,1 g Kaliumacetat in 392 ml Wasser getränkt und mit Heißluft bei 150°C getrocknet.

Der fertige Katalysator enthielt 1,0 Gew.-% Pd, 0,4 Gew.-% Au und 2,8 Gew.-% K; die Schalendicke betrug 0,7 mm.

Die Austestung erfolgte wie in Vergleichsbeispiel 4; die Ergebnisse sind aus der Tabelle 2 ersichtlich.

**Tabelle 1**

| | Leistung [g/lh] | spez. Leistung (*) | Vinylacetatgehalt (Gew.-%) im kondensierten Reaktorausgangsgas | Selektivität [%] |
|---|---|---|---|---|
| Vergleichsbeispiel 1 (Pd/K/Cd □ ) | 813 | 70,7 | 25,7 | 94,3 |
| Vergleichsbeispiel 2 (Pd/K/Ba □ ) | 827 | 71,9 | 25,9 | 92,8 |
| Vergleichsbeispiel 3 (Pd/K/Cd #) | 735 | 98,0 | 24,0 | 91,4 |
| Vergleichsbeispiel 4 (Pd/K/Au #) | 710 | 142,0 | 22,6 | 89,3 |

| | | | | |
|---|---|---|---|---|
| * Gramm Vinylacetat pro Gramm Palladium pro Stunde | | | | |
| □ durchimprägniert | | | | |
| # Schalenkatalysator gemäß deutscher Patentanmeldung P 41 20 492.1 | | | | |

**Tabelle 2**

| | Leistung [g/lh] | spez. Leistung (*) | Vinylacetatgehalt (%) im kondensierten Reaktorausgangsgas | Selektivität [%] |
|---|---|---|---|---|
| Beispiel 1a (Pd/K/Cd) | 760 | 108,6 | 25,0 | 98,0 |
| Beispiel 1b (Pd/K/Cd) | 915 | 79,6 | 33,0 | 96,3 |
| Beispiel 2 (Pd/K/Ba) | 917 | 79,7 | 33,1 | 95,7 |
| Beispiel 3 (Pd/K/Au) | 740 | 148,0 | 24,2 | 90,0 |

| | | | | |
|---|---|---|---|---|
| * Gramm Vinylacetat pro Gramm Palladium pro Stunde | | | | |

## Patentansprüche

1. Verfahren zur Herstellung eines Palladium, Kalium und Cadmium enthaltenden Trägerkatalysators, dadurch gekennzeichnet, daß man Salze des Palladiums, Kaliums und Cadmiums in einem geeigneten Lösungsmittel löst, die Lösung durch Ultraschall zerstäubt und mit der zerstäubten Lösung das Trägermaterial ein- oder mehrmals imprägniert und nach jeder Imprägnierung trocknet, wobei das Lösungsvolumen bei jeder Imprägnierung mindestens 5 bis höchstens 60 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 5 bis höchstens 60 % des Volumens der Trägerteilchen die genannten Salze enthält.

2. Verfahren nach Anspruch 1, dadurch gekennzeichnet, daß das Lösungsvolumen bei jeder Imprägnierung mit den Pd-, K- und Cd-Salzen mindestens 15 bis höchstens 40 % des Porenvolumens beträgt, wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 15 bis höchstens 40 % des Volumens der Trägerteilchen die genannten Salze enthält.

3. Palladium, Kalium und Cadmium enthaltender Trägerkatalysator, dadurch erhältlich, daß man Salze des Palladiums, Kaliums und Cadmiums in einem geeigneten Lösungsmittel löst, die Lösung durch Ultraschall zerstäubt und mit der zerstäubten Lösung das Trägermaterial ein- oder mehrmals imprägniert und nach jeder Imprägnierung trocknet, wobei das Lösungsvolumen bei jeder Imprägnierung mindestens 5 bis höchstens 60 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 5 bis höchstens 60 % des Volumens der Trägerteilchen die genannten Salze enthält.

4. Trägerkatalysator nach Anspruch 3, dadurch erhältlich, daß das Lösungsvolumen bei jeder Imprägnierung mit den Pd-, K- und Cd-Salzen mindestens 15 bis höchstens 40 % des Porenvolumens beträgt, wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 15 bis höchstens 40 % des Volumens der Trägerteilchen die genannten Salze enthält.

5. Verwendung des nach Anspruch 1 erhältlichen Trägerkatalysators zur Herstellung von Vinylacetat in der Gasphase aus Ethylen, Essigsäure und Sauerstoff oder Sauerstoff enthaltenden Gasen.

6. Verwendung des nach Anspruch 2 erhältlichen Trägerkatalysators zur Herstellung von Vinylacetat in der Gasphase aus Ethylen, Essigsäure und Sauerstoff oder Sauerstoff enthaltenden Gasen.

7. Verfahren zur Herstellung eines Palladium, Kalium und Barium enthaltenden Trägerkatalysators, dadurch gekennzeichnet, daß man Salze des Palladiums, Kaliums und Bariums in einem geeigneten Lösungsmittel löst, die Lösung durch Ultraschall zerstäubt und mit der zerstäubten Lösung das Trägermaterial ein- oder mehrmals imprägniert und nach jeder Imprägnierung trocknet, wobei das Lösungsvolumen bei jeder Imprägnierung mindestens 15 bis höchstens 80 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 15 bis höchstens 80 % des Volumens der Trägerteilchen die genannten Salze enthält.

8. Verfahren nach Anspruch 7, dadurch gekennzeichnet, daß das Lösungsvolumen bei jeder Imprägnierung mit den Pd-, K- und Ba-Salzen mindestens 25 bis höchstens 40 % des Porenvolumens des Trägermaterials beträgt, wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 25 bis höchstens 40 % des Volumens der Trägerteilchen die genannten Salze enthält.

9. Palladium, Kalium und Barium enthaltender Trägerkatalysator, dadurch erhältlich, daß man Salze des Palladiums, Kaliums und Bariums in einem geeigneten Lösungsmittel löst, die Lösung durch Ultraschall zerstäubt und mit der zerstäubten Lösung das Trägermaterial ein- oder mehrmals imprägniert und nach jeder Imprägnierung trocknet, wobei das Lösungsvolumen bei jeder Imprägnierung mindestens 15 bis höchstens 80 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 15 bis höchstens 80 % des Volumens der Trägerteilchen die genannten Salze enthält.

10. Trägerkatalysator nach Anspruch 9, dadurch erhältlich, daß das Lösungsvolumen bei jeder Imprägnierung mit den Pd-, K- und Ba-Salzen mindestens 25 bis höchstens 40 % des Porenvolumens des Trägermaterials beträgt, wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 25 bis höchstens 40 % des Volumens der Trägerteilchen die genannten Salze enthält.

11. Verwendung des nach Anspruch 7 erhältlichen Trägerkatalysators zur Herstellung von Vinylacetat in der Gasphase aus Ethylen, Essigsäure und Sauerstoff oder Sauerstoff enthaltenden Gasen.

12. Verwendung des nach Anspruch 8 erhältlichen Trägerkatalysators zur Herstellung von Vinylacetat in der Gasphase aus Ethylen, Essigsäure und Sauerstoff oder Sauerstoff enthaltenden Gasen.

13. Verfahren zur Herstellung eines Palladium, Kalium und Gold enthaltenden Trägerkatalysators, dadurch gekennzeichnet, daß man Salze des Palladiums, Kaliums und Golds in einem geeigneten Lösungsmittel löst, die Lösung durch Ultraschall zerstäubt und mit der zerstäubten Lösung das Trägermaterial ein-oder mehrmals imprägniert und nach jeder Imprägnierung trocknet, wobei das Lösungsvolumen bei jeder Imprägnierung mindestens 5 bis höchstens 80 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 5 bis höchstens 80 % des Volumens der Trägerteilchen die genannten Salze enthält.

14. Verfahren nach Anspruch 13, dadurch gekennzeichnet, daß das Lösungsvolumen bei jeder Imprägnierung mit den Pd-, K- und Au-Salzen mindestens 15 bis höchstens 50 % des Porenvolumens des Trägermaterials beträgt wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 15 bis höchstens 50 % des Volumens der Trägerteilchen die genannten Salze enthält.

15. Palladium, Kalium und Gold enthaltender Trägerkatalysator, dadurch erhältlich, daß man Salze des Palladiums, Kaliums und Golds in einem geeigneten Lösungsmittel löst, die Lösung durch Ultraschall zerstäubt und mit der zerstäubten Lösung das Trägermaterial ein- oder mehrmals imprägniert und nach jeder Imprägnierung trocknet, wobei das Lösungsvolumen bei jeder Imprägnierung mindestens 5 bis höchstens 80 % des Porenvolumens des Trägermaterials beträgt und die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 5 bis höchstens 80 % des Volumens der Trägerteilchen die genannten Salze enthält.

16. Trägerkatalysator nach Anspruch 15, dadurch erhältlich, daß das Lösungsvolumen bei jeder Imprägnierung mit den Pd-, K- und Au-Salzen mindestens 15 bis höchstens 50 % des Porenvolumens des Trägermaterials beträgt wobei die Dauer jeder Imprägnierung sowie die Zeit bis zum Beginn der auf diese Imprägnierung folgenden Trocknung so kurz gewählt werden, daß nach Ende der letzten Trocknung eine Schale von mindestens 15 bis höchstens 50 % des Volumens der Trägerteilchen die genannten Salze enthält.

17. Verwendung des nach Anspruch 13 erhältlichen Trägerkatalysators zur Herstellung von Vinylacetat in der Gasphase aus Ethylen, Essigsäure und Sauerstoff oder Sauerstoff enthaltenden Gasen.

18. Verwendung des nach Anspruch 14 erhältlichen Trägerkatalysators zur Herstellung von Vinylacetat in der Gasphase aus Ethylen, Essigsäure und Sauerstoff oder Sauerstoff enthaltenden Gasen.

## Claims

1. A process for the preparation of a supported catalyst containing palladium, potassium and cadmium, which comprises dissolving salts of palladium, of potassium and of cadmium in a suitable solvent, atomising the solution by ultrasonics, impregnating the carrier material once or repeatedly with the atomized solution and drying it after each impregnation, the solution volume for each impregnation being at least 5 to at most 60% of the pore volume of the carrier material and the duration of each impregnation and the time up to the beginning of the drying which follows this impregnation being chosen to be so short that, after the end of the final drying, a coat of at least 5 to at most 60% of the volume of the carrier particles contains the stated salts.

2. The process as claimed in claim 1, wherein the solution volume for each impregnation with the Pd, K and Cd salts is at least 15 to at most 40% of the pore volume, the duration of each impregnation and the time up to the beginning of the drying which follows this impregnation being chosen to be so short that, after the end of the final drying, a coat of at least 15 to at most 40% of the volume of the carrier particles contains the stated salts.

3. A supported catalyst containing palladium, potassium and cadmium, obtainable by dissolving salts of palladium, of potassium and of cadmium in a suitable solvent, atomizing the solution by ultrasonics, impregnating the carrier material once or repeatedly with the atomized solution and drying it after each impregnation, the solution volume for each impregnation being at least 5 to at most 60% of the pore volume of the carrier material and the duration of each impregnation and the time up to the beginning of the drying which follows this impregnation being chosen to be so short that, after the end of the final drying, a coat of at least 5 to at most 60% of the volume of the carrier particles contains the stated salts.

4. A supported catalyst as claimed in claim 3, obtainable by a method in which the solution volume for each impregnation with the Pd, K and Cd salts is at least 15 to at most 40% of the pore volume, the duration of each impregnation and the time up to the beginning of the drying which follows this impregnation being chosen to be so short that, after the end of the final drying, a coat of at least 15 to at most 40% of the volume of the carrier particles contains the stated salts.

5. The use of the supported catalyst obtainable as claimed in claim 1 for the preparation of vinyl acetate in the gas phase from ethylene, acetic acid and oxygen or oxgyen-containing gases.

6. The use of the supported catalyst obtainable as claimed in claim 2 for the preparation of vinyl acetate in the gas phase from ethylene, acetic acid and oxygen or oxgyen-containing gases.

7. A process for the preparation of a supported catalyst containing palladium, potassium and barium, which comprises dissolving salts of palladium, of potassium and of barium in a suitable solvent, atomizing the solution by ultrasonics, impregnating the carrier material once or repeatedly with the atomized solution and drying it after each impregnation, the solution volume for each impregnation being at least 15 to at most 80% of the pore volume of the carrier material and the duration of each impregnation and the time up to the beginning of the drying which follows this impregnation being chosen to be so short that, after the end of the final drying, a coat of at least 15 to at most 80% of the volume of the carrier particles contains the stated salts.

8. The process as claimed in claim 7, wherein the solution volume for each impregnation with the Pd, K and Ba salts is at least 25 to at most 40% of the pore volume of the carrier material, the duration of each impregnation and the time up to the beginning of the drying which follows this impregnation being chosen to be so short that, after the end of the final drying, a coat of at least 25 to at most 40% of the volume of the carrier particles contains the stated salts.

9. A supported catalyst containing palladium, potassium and barium, obtainable by dissolving salts of palladium, of potassium and of barium in a suitable solvent, atomizing the solution by ultrasonics, impregnating the carrier material once or repeatedly with the atomized solution and drying it after each impregnation, the solution volume for each impregnation being at least 15 to at most 80% of the pore volume of the carrier material and the duration of each impregnation and the time up to the beginning of the drying which follows this impregnation being chosen to be so short that, after the end of the final drying, a coat of at least 15 to at most 80% of the volume of the carrier particles contains the stated salts.

10. A supported catalyst as claimed in claim 9, obtainable by a method in which the solution volume for each impregnation with the Pd, K and Ba salts is at least 25 to at most 40% of the pore volume of the carrier material, the duration of each impregnation and the time up to the beginning of the drying which follows this impregnation being chosen to be so short that, after the end of the final drying, a coat of at least 25 to at most 40% of the volume of the carrier particles contains the stated salts.

11. The use of the supported catalyst obtainable as claimed in claim 7 for the preparation of vinyl acetate in the gas phase from ethylene, acetic acid and oxygen or oxgyen-containing gases.

12. The use of the supported catalyst obtainable as claimed in claim 8 for the preparation of vinyl acetate in the gas phase from ethylene, acetic acid and oxygen or oxgyen-containing gases.

13. A process for the preparation of a supported catalyst containing palladium, potassium and gold, which comprises dissolving salts of palladium, of potassium and of gold in a suitable solvent, atomizing the solution by ultrasonics, impregnating the carrier material once or repeatedly with the atomized solution and drying it after each impregnation, the solution volume for each impregnation being at least 5 to at most 80% of the pore volume of the carrier material and the duration of each impregnation and the time up to the beginning of the drying which follows this impregnation being chosen to be so short that, after the end of the final drying, a coat of at least 5 to at most 80% of the volume of the carrier particles contains the stated salts.

14. The process as claimed in claim 13, wherein the solution volume for each impregnation with the Pd, K and Au salts is at least 15 to at most 50% of the pore volume of the carrier material, the duration of each impregnation and the time up to the beginning of the drying which follows this impregnation being chosen to be so short that, after the end of the final drying, a coat of at least 15 to at most 50% of the volume of the carrier particles contains the stated salts.

15. A supported catalyst containing palladium, potassium and gold, obtainable by dissolving salts of palladium, of potassium and of gold in a suitable solvent, atomizing the solution by ultrasonics, impregnating the carrier material once or repeatedly with the atomized solution and drying it after each impregnation, the solution volume for each impregnation being at least 5 to at most 80% of the pore volume of the carrier material and the duration of each impregnation and the time up to the beginning of the drying which follows this impregnation being chosen to be so short that, after the end of the final drying, a coat of at least 5 to at most 80% of the volume of the carrier particles contains the stated salts.

16. A supported catalyst as claimed in claim 15, obtainable by a method in which the solution volume for each impregnation with the Pd, K and Au salts is at least 15 to at most 50% of the pore volume of the carrier material, the duration of each impregnation and the time up to the beginning of the drying which follows this impregnation being chosen to be so short that, after the end of the final drying, a coat of at least 15 to at most 50% of the volume of the carrier particles contains the stated salts.

17. The use of the supported catalyst obtainable as claimed in claim 13 for the preparation of vinyl acetate in the gas phase from ethylene, acetic acid and oxygen or oxgyen-containing gases.

18. The use of the supported catalyst obtainable as claimed in claim 14 for the preparation of vinyl acetate in the gas phase from ethylene, acetic acid and oxygen or oxgyen-containing gases.

## Revendications

1. Procédé de préparation d'un catalyseur supporté contenant du palladium, du potassium et du cadmium, caractérisé en ce que l'on dissout des sels de palladium, de potassium et de cadmium dans un solvant approprié, on pulvérise la solution à l'aide d'ultrasons, et on imprègne le support une ou plusieurs fois avec la solution pulvérisée et on le sèche après chaque imprégnation, le volume de solution pour chaque imprégnation étant compris entre au moins 5 et au plus 60 % du volume des pores du support, et la durée de chaque imprégnation ainsi que le temps s'écoulant jusqu'au début du séchage suivant cette imprégnation étant choisis assez courts pour que, à la fin du dernier séchage, lesdits sels soient contenus dans une couche représentant au moins 5 à au plus 60 % du volume des particules de support.

2. Procédé selon la revendication 1, caractérisé en ce que, pour chaque imprégnation avec les sels de Pd, K et Cd, le volume de solution est compris entre au moins 15 et au plus 40 % du volume des pores, la durée de chaque imprégnation et le temps s'écoulant jusqu'au début du séchage suivant cette imprégnation étant choisis assez courts pour que, à la fin du dernier séchage, lesdits sels soient contenus dans une couche représentant au moins 15 à au plus 40 % du volume des particules de support.

3. Catalyseur supporté contenant du palladium, du potassium et du cadmium, pouvant être obtenu par un procédé selon lequel on dissout des sels de palladium, de potassium et de cadmium dans un solvant approprié, on pulvérise la solution à l'aide d'ultrasons, et on imprègne le support une ou plusieurs fois avec la solution pulvérisée et on le sèche après chaque imprégnation, le volume de solution pour chaque imprégnation étant compris entre au moins 5 et au plus 60 % du volume des pores du support, et la durée de chaque imprégnation ainsi que le temps s'écoulant jusqu'au début du séchage suivant cette imprégnation étant choisis assez courts pour que, à la fin du dernier séchage, lesdits sels soient contenus dans une couche représentant au moins 5 à au plus 60 % du volume des particules de support.

4. Catalyseur supporté selon la revendication 3, pouvant être obtenu par un procédé selon lequel, pour chaque imprégnation avec les sels de Pd, K et Cd, le volume de solution est compris entre au moins 15 et au plus 40 % du volume des pores, la durée de chaque imprégnation et le temps s'écoulant jusqu'au début du séchage suivant cette imprégnation étant choisis assez courts pour que, à la fin du dernier séchage, lesdits sels soient contenus dans une couche représentant au moins 15 à au plus 40 % du volume des particules de support.

5. Utilisation du catalyseur supporté pouvant être obtenu selon la revendication 1 pour la préparation d'acétate de vinyle en phase gazeuse à partir d'éthylène, d'acide acétique et d'oxygène ou de gaz contenant de l'oxygène.

6. Utilisation du catalyseur supporté pouvant être obtenu selon la revendication 2 pour la préparation d'acétate de vinyle en phase gazeuse à partir d'éthylène, d'acide acétique et d'oxygène ou de gaz contenant de l'oxygène.

7. Procédé de préparation d'un catalyseur supporté contenant du palladium, du potassium et du baryum, caractérisé en ce que l'on dissout des sels de palladium, de potassium et de baryum dans un solvant approprié, on pulvérise la solution à l'aide d'ultrasons, et on imprègne le support une ou plusieurs fois avec la solution pulvérisée et on le sèche après chaque imprégnation, le volume de solution pour chaque imprégnation étant compris entre au moins 15 et au plus 80 % du volume des pores du support, et la durée de chaque imprégnation ainsi que le temps s'écoulant jusqu'au début du séchage suivant cette imprégnation étant choisis assez courts pour que, à la fin du dernier séchage, lesdits sels soient contenus dans une couche représentant au moins 15 à au plus 80 % du volume des particules de support.

8. Procédé selon la revendication 7, caractérisé en ce que, pour chaque imprégnation avec les sels de Pd, K et Ba, le volume de solution est compris entre au moins 25 et au plus 40 % du volume des porcs du support, la durée de chaque imprégnation et le temps s'écoulant jusqu'au début du séchage suivant cette imprégnation étant choisis assez courts pour que, à la fin du dernier séchage, lesdits sels soient contenus dans une couche représentant au moins 25 à au plus 40 % du volume des particules de support.

9. Catalyseur supporté contenant du palladium, du potassium et du baryum, pouvant être obtenu par un procédé selon lequel on dissout des sels de palladium, de potassium et de baryum dans un solvant approprié, on pulvérise la solution à l'aide d'ultrasons, et on imprègne le support une ou plusieurs fois avec la solution pulvérisée et on le sèche après chaque imprégnation, le volume de solution pour chaque imprégnation étant compris entre au moins 15 et au plus 80 % du volume des pores du support, et la durée de chaque imprégnation ainsi que le temps s'écoulant jusqu'au début du séchage suivant cette imprégnation étant choisis assez courts pour que, à la fin du dernier séchage, lesdits sels soient contenus dans une couche représentant au moins 15 à au plus 80 % du volume des particules de support.

10. Catalyseur supporté selon la revendication 9, pouvant être obtenu par un procédé selon lequel, pour chaque imprégnation avec les sels de Pd, K et Ba, le volume de solution est compris entre au moins 25 et au plus 40 % du volume des pores du support, la durée de chaque imprégnation et le temps s'écoulant jusqu'au début du séchage suivant cette imprégnation étant choisis assez courts pour que, à la fin du dernier séchage, lesdits sels soient contenus dans une couche représentant au moins 25 à au plus 40 % du volume des particules de support.

11. Utilisation du catalyseur supporté pouvant être obtenu selon la revendication 7 pour la préparation d'acétate de vinyle en phase gazeuse à partir d'éthylène, d'acide acétique et d'oxygène ou de gaz contenant de l'oxygène.

12. Utilisation du catalyseur supporté pouvant être obtenu selon la revendication 8 pour la préparation d'acétate de vinyle en phase gazeuse à partir d'éthylène, d'acide acétique et d'oxygène ou de gaz contenant de l'oxygène.

13. Procédé de préparation d'un catalyseur supporté contenant du palladium, du potassium et de l'or, caractérisé en ce que l'on dissout des sels de palladium, de potassium et d'or dans un solvant approprié, on pulvérise la solution à l'aide d'ultrasons, et on imprègne le support une ou plusieurs fois avec la solution pulvérisée et on le sèche après chaque imprégnation, le volume de solution pour chaque imprégnation étant compris entre au moins 5 et au plus 80 % du volume des pores du support, et la durée de chaque imprégnation ainsi que le temps s'écoulant jusqu'au début du séchage suivant cette imprégnation étant choisis assez courts pour que, à la fin du dernier séchage, lesdits sels soient contenus dans une couche représentant au moins 5 à au plus 80 % du volume des particules de support.

14. Procédé selon la revendication 13, caractérisé en ce que, pour chaque imprégnation avec les sels de Pd, K et Au, le volume de solution est compris entre au moins 15 et au plus 50 % du volume des porcs du support, la durée de chaque imprégnation et le temps s'écoulant jusqu'au début du séchage suivant cette imprégnation étant choisis assez courts pour que, à la fin du dernier séchage, lesdits sels soient contenus dans une couche représentant au moins 15 à au plus 50 % du volume des particules de support.

15. Catalyseur supporté contenant du palladium, du potassium et de l'or, pouvant être obtenu par un procédé selon lequel on dissout des sels de palladium, de potassium et d'or dans un solvant approprié, on pulvérise la solution à l'aide d'ultrasons, et on imprègne le support une ou plusieurs fois avec la solution pulvérisée et on le sèche après chaque imprégnation, le volume de solution pour chaque imprégnation étant compris entre au moins 5 et au plus 80 % du volume des pores du support, et la durée de chaque imprégnation ainsi que le temps s'écoulant jusqu'au début du séchage suivant cette imprégnation étant choisis assez courts pour que, à la fin du dernier séchage, lesdits sels soient contenus dans une couche représentant au moins 5 à au plus 80 % du volume des particules de support.

16. Catalyseur supporté selon la revendication 15, pouvant être obtenu par un procédé selon lequel, pour chaque imprégnation avec les sels de Pd, K et Au, le volume de solution est compris entre au moins 15 et au plus 50 % du volume des pores du support, la durée de chaque imprégnation et le temps s'écoulant jusqu'au début du séchage suivant cette imprégnation étant choisis assez courts pour que, à la fin du dernier séchage, lesdits sels soient contenus dans une couche représentant au moins 15 à au plus 50 % du volume des particules de support.

17. Utilisation du catalyseur supporté pouvant être obtenu selon la revendication 13 pour la préparation d'acétate de vinyle en phase gazeuse à partir d'éthylène, d'acide acétique et d'oxygène ou de gaz contenant de l'oxygène.

18. Utilisation du catalyseur supporté pouvant être obtenu selon la revendication 14 pour la préparation d'acétate de vinyle en phase gazeuse à partir d'éthylène, d'acide acétique et d'oxygène ou de gaz contenant de l'oxygène.
